# EUROPÄISCHE PATENTSCHRIFT

(11) **EP 2 981 509 B1**
(45) Veröffentlichungstag und Bekanntmachung des Hinweises auf die Patenterteilung: **08.11.2017**
(21) Anmeldenummer: 14715867.9
(22) Anmeldetag: 27.03.2014
(51) Int. Cl.: C03C 3/097, C03C 4/00, C03C 10/00, A61K 6/00, A61K 6/02, A61K 6/027, A61K 6/033

(54) **HOCHFESTE, TRANSLUZENTE MG-HOCHQUARZMISCHKRISTALL-GLASKERAMIK**
HIGH-STRENGTH, TRANSLUCENT MG-HIGH QUARTZ MIXED CRYSTAL GLASS CERAMICS
VITROCÉRAMIQUE À BASE DE CRISTAUX MIXTES DE QUARTZ BÊTA-MG, TRANSLUCIDE DE HAUTE RÉSISTANCE

(30) Priorität: 02.04.2013 DE 102013103285; 31.07.2013 DE 102013108216
(43) Veröffentlichungstag der Anmeldung: 10.02.2016
(73) Patentinhaber: SIRONA Dental Systems GmbH, 64625 Bensheim (DE)
(72) Erfinder: DURSCHANG, Bernhard, 97228 Rottendorf (DE); PROBST, Jörn, 97273 Kürnach (DE); KATZSCHMANN, Axel, 68723 Schwetzingen (DE)
(74) Vertreter: Sommer, Peter
(86) Internationale Anmeldenummer: PCT/EP2014/056202
(87) Internationale Veröffentlichungsnummer: WO 2014/161770

(56) Entgegenhaltungen:
- DE-A1-102010 050 275
- JP-A- 2001 026 441
- MARC DITTMER ET AL: "Colorless and high strength MgO/Al2O3/SiO2 glass-ceramic dental material using zirconia as nucleating agent", JOURNAL OF BIOMEDICAL MATERIALS RESEARCH PART B: APPLIED BIOMATERIALS, Bd. 100B, Nr. 2, 21. November 2011 (2011-11-21), Seiten 463-470, XP055121574, ISSN: 1552-4973, DOI: 10.1002/jbm.b.31972
- Christian Rüssel ET AL: "Zahnersatz aus hochfesten Glaskeramiken", , 3. Dezember 2012 (2012-12-03), XP055121565, Gefunden im Internet: URL:http://www.git-labor.de/forschung/mate rialien/zahnersatz-aus-hochfesten-glaskera miken [gefunden am 2014-06-04] in der Anmeldung erwähnt

## Beschreibung

Die Erfindung betrifft Glaskeramiken auf Basis des Hochquarzmischkristall-Systems, die in einer Zwischenstufe der Kristallisation einfach mechanisch bearbeitbar sind und nach der vollständigen Kristallisation eine hochfeste, hoch transluzente und chemisch stabile Glaskeramik darstellen.

Hochquarzmischkristall-Glaskeramiken sind aus der Literatur gut bekannt; eine Reihe von Patent-Veröffentlichungen betreffen dieses Glaskeramiksystem. Hauptanwendungsgebiete der dort genannten Zusammensetzungen sind hochfeste Substrate für Informationsaufzeichnungsmedien (z. B. Substrate für Magnetspeicherplatten), Kochplatten, Spiegelsubstrate und Brandschutzmaterialien (z. B. Kaminfenster), wie z. B. in EP 1 391 438 A1, DE 199 39 787 A1, DE 10 2004 024583 A1 und EP 1 029 830 A1 angegeben.

Alle diese Patente basieren auf einem System, das aus den Hauptkomponenten Lithiumoxid, Aluminiumoxid und Siliciumoxid besteht. Dieses zeichnet sich insbesondere durch seine minimalen bis negativen Wärmeausdehnungskoeffizienten aus.

DE 20 2004 009 227 U1 schlägt eine Glas-/Glaskeramik/Metall-Durchführung für eine Leuchtvorrichtung mit einem Glas- oder Glaskeramikkörper vor, wobei der Körper aus einer Li₂O-Al₂O₃SiO₂-Glaskeramik bestehen kann, die MgO, TiO₂ und/oder ZrO₂ und fakultativ weiterhin P₂O₅ enthalten kann.

In DE 103 46 197 A1 ist eine Glaskeramik angegeben, die sich als Substrat für optische und elektronische Bauteile eignet und SiO₂, B₂O₃, Al₂O₃, MgO, TiO₂, ZrO₂ und fakultativ auch P₂O₅ enthält.

Neben den Lithium-Hochquarzmischkristall-Glaskeramiken sind auch Magnesium-Hochquarzmischkristalle bekannt. Hier wären zu nennen: DE 26 02 429 C2, US 6,953,756 B2 und US 2008/0207425 A1. Für dentale Anwendungen, insbesondere für CAD/CAM bearbeitbare Glaskeramiken, ist auf den Artikel "Zahnersatz aus hochfesten Glaskeramiken" zu verweisen, verfügbar im Internet unter www. git-labor.de/forschung/materialien/zahnersatz-aushochfesten-glaskeramiken.

M. Dittmer und C. Rüssel, Journal of Biomedical Materials Research Part B: Applied Biomaterials, Bd. 100B(2), Seiten 463-470 (2011) beschreiben farblose MgO/Al₂O₃/SiO₂-Glaskeramiken für dentale Zwecke, denen Zirkoniumdioxid als Nucleierungsmittel zugesetzt wird. Einige der Zusammensetzungen enthalten weiterhin P₂O₅.

In JP 2001-02644A wird eine für magnetische Scheiben aus Glaskeramik geeignete Zusammensetzung offenbart, die aus 42,5 MA SiO₂, 27 MA Al₂O₃, 16,5 MA MgO, 5 MA P₂O₅ und 9 MA TiO₂ besteht.

CAD/CAM bearbeitbare Glaskeramiken zeichnen sich ganz generell dadurch aus, dass das entsprechende Material in einer Zwischenstufe mit geringer Festigkeit mittels CAM gut bearbeitet und anschließend durch eine kurze thermische Nachbearbeitung in eine hochfeste Glaskeramik überführt werden kann. Solche Glaskeramiken sind aus dem Lithiumsilicat-System bekannt. Hier zeigt z. B. bereits DE-A-24 51 121 aus dem Jahre 1974, dass Glaskeramiken, die Lithiummetasilicat als Hauptphase enthalten, eine verringerte Festigkeit im Vergleich zu Glaskeramiken haben, die Lithiumdisilicat als einzige kristalline Phase enthalten. Dieses Prinzip wurde ausgenutzt, um in einem zweistufigen Kristallisationsprozess zuerst eine Glaskeramik herzustellen, die mechanisch gut bearbeitbar ist, z. B. mittels CAD/CAM-Verfahren, und diese anschließend in einer zweiten Kristallisationsstufe zur Dentalglaskeramik zu prozessieren. Dieses Verfahren ist geeignet, um dentale Restaurationen nach dem sogenannten chair-side-Verfahren verwenden zu können. Bei diesem Verfahren wird in der Zahnarztpraxis aus einem Glaskeramikblock nach der ersten Kristallisationsstufe mittels CAD/CAM eine individuell angepasste Krone/Onlay/Inlay herausgefräst, diese in einem Spezialofen der zweiten Kristallisationsstufe unterzogen und direkt in der ersten und einzigen Zahnarztsitzung dem Patienten eingesetzt, siehe z.B. DE 10 2005 028637 A1. Mit einem Zusatz an Stabilisatoren wie Zirkoniumoxid konnten die Glaskeramiken hinsichtlich Festigkeit und Transluzenz nochmals verbessert werden, siehe WO 2012/059143.

Es ist allerdings wünschenswert, Glaskeramiken für z.B. dentale Zwecke bereitzustellen, die nicht nur eine hohe Festigkeit besitzen, sondern außerdem ein derartiges Gefüge aufweisen, dass eine Rissausbreitung durch Mikrospannungen möglichst weitgehend verhindert wird. Derartige Mikrospannungen können beispielsweise im Gefolge der Temperschritte auftreten, die für die mehrstufige Herstellung der Glaskeramiken eingesetzt werden müssen.

Bei dieser Überlegung ansetzend haben sich die Erfinder das weitere Ziel gesetzt, dass eine Glaskeramik, die diese wünschenswerten Eigenschaften besitzen sollte, zusätzlich möglichst lithiumarm oder lithiumfrei sein sollte. Denn Lithiumsalze werden seit über 60 Jahren für die Behandlung von affektiven Störungen wie bipolaren Erkrankungen oder Depressionen eingesetzt. Die physiologische Wirkung der Lithiumsalze ist dabei weitgehend unbekannt, da diese auf zahllose Prozesse im menschlichen Körper einwirken. Möglicherweise vermindert Lithium die Wahrscheinlichkeit einer weiteren affektiven Episode, indem es bei manischen Episoden einen Noradrenalinüberschuss senkt und bei depressiven Episoden die Serotoninproduktion aktiviert. Dabei ist die therapeutische Breite von Lithium gering, das heißt: Auch ein nur geringfügiger Anstieg an Lithiumionen im Körper über die therapeutisch üblicherweise eingesetzte Menge hinaus kann zusätzliche und gefährliche Nebenwirkungen hervorrufen. Nun sind Lithiumkationen aufgrund ihrer Größe relativ leicht löslich und könnten in feuchter Umgebung möglicherweise auch aus einem Verbund mit Silikat- und Phosphatanionen langfristig zumindest in sehr kleinen Mengen austreten. Ob dies tatsächlich stattfindet und darüber hinaus im Falle von dentalen Anwendungen problematisch sein könnte, ist unbekannt. Dennoch wäre es deshalb von Vorteil, wenn Glaskeramiken zur Verfügung stünden, die arm an oder frei von Lithiumionen sind.

Die vorliegende Erfindung betrifft ein Verfahren zum Herstellen einer Glaskeramik des Systems MgO-Al₂O₃-SiO₂ die einerseits nur geringe Mengen an Lithiumoxid enthält oder frei von Lithiumoxid ist, andererseits aber signifikante Mangen an Phosphoroxid aufweist. Wird aus diesem Material ein Glas erschmolzen und in ein oder zwei Schritten auf eine Temperatur von maximal 900°C gebracht, erhält man ein kristallhaltiges Material, das sich z.B. mittels CAD/CAM noch gut bearbeiten lässt. Wird dieses in einer nachfolgenden Stufe auf über 900°C erhitzt, entsteht ein hochfestes Material, das hochtransparent ist und auch eine bessere chemische Beständigkeit besitzt als bekannte dentale Glaskeramiken.

Bei den nachstehenden Erläuterungen ist zu beachten, dass die Masse-Angaben für die jeweiligen Kationen immer in Bezug auf eine reine Oxid-Keramik angegeben sind; sofern die Glaskeramik auch andere Anionen enthält, sind entsprechende Korrekturen vorzunehmen. Die Angaben erfolgen in Masse-%, entsprechend Masse-Anteilen (MA) auf hundert Teile Zusammensetzung.

Glaskeramiken aus dem System Magnesiumoxid-Aluminiumoxid-Siliciumdioxid (MAS) sind für ihre guten mechanischen Eigenschaften, insbesondere ihre hohen Festigkeiten, bekannt. Dieses System dient auch der vorliegenden Erfindung als Ausgangssystem. Dabei sind die drei Komponenten in breiten Bereichen variierbar. Insbesondere kann bereits ein recht kleiner Anteil an MgO von etwa 1 Masse-% ausreichend sein; dieser Anteil kann jedoch auch bis auf 20 Masse-% steigen. Der Anteil an Al₂O₃ kann zwischen ca. 12 und 30 Masse-% schwanken; der Anteil an SiO₂ kann bei 30 bis 60 Masse-% liegen. Meist liegt der Anteil an MgO bei über 5 Masse-%; der Anteil an Al₂O₃ beträgt häufig nicht weniger als 15 Masse-%. Der Anteil an SiO₂ liegt häufig im Bereich zwischen 35 und 55 Masse-%. Bevorzugte Bereiche sind: MgO-Anteil 6,0 bis 14,5 Masse-%; Al₂O₃-Anteil 16 bis 27 Masse-%; SiO₂-Anteil 40 bis 55 Masse-%.

Es ist weiterhin wünschenswert, dass die Keramik kaum oder kein Lithium enthält. Im Sinne der Erfindung muss der Anteil unter 5 Masse-% Li₂O liegen; vorzugsweise liegt er unter 2 Masse-%, Stärker bevorzugt sind nur Spuren (< 1 Masse-% bis 0 Masse-%) an Li₂O vorhanden, die z.B. unbeabsichtigt eingeschleppt wurden.

Es ist im Stand der Technik bekannt, Phosphor zu Lithiumsilikat-Glaskeramiken als Keimbildner zuzusetzen. Völlig überraschend hat sich herausgestellt, dass der Zusatz von Phosphor in den vorliegenden, lithiumfreien oder lithiumarmen MAS-Glaskeramiken der Ausbreitung von Mikrorissen entgegenwirkt, wenn spezifische Bedingungen für die Aushärtung der zweiten Stufe eingehalten werden, die nachstehend näher erläutert werden. Initial kristallisiert der Phosphor dabei in einer eigenen, phosphorhaltigen Phase, die die Bildung einer Vielzahl relativ kleiner MAS-Kristalle bewirkt, die als β-Quarz auskristallisieren. Dadurch erhöht sich die Anzahl der Kristalle, die in der final ausgehärteten Glaskeramik jedoch relativ klein (< 2 µm) bleiben, was einerseits eine hohe Festigkeit bewirkt, wodurch andererseits aber die bereits oben angesprochene Rissausbreitung in günstiger Weise unterdrückt wird. Des Weiteren bewirken die kleinen Kristallite eine hohe Transluzenz.

Der Anteil an Phosphor, ausgedrückt in P₂O₅, sollte zu diesem Zweck nicht unter 3,5 Masse-% liegen. Bevorzugt ist ein Bereich von 5 bis 15 Masse-%, stärker bevorzugt ein Bereich von 7,5 bis 15 Masse-%. Höhere Anteile sind insbesondere dann günstig, wenn der Anteil an MgO relativ gering ist und umgekehrt.

Die Zusammensetzung enthält weiterhin substantielle Anteile an einer Übergangsmetalloxid-Komponente, ausgewählt unter TiO₂ und ZrO₂. Diese beiden Substanzen sollten zusammen in einem Anteil von mindestens 5 Masse-%, stärker bevorzugt 8 Masse-% enthalten sein und können in einem Anteil von bis zu ca. 20 Masse-%, vorzugsweise bis ca. 15 Masse-% vorliegen. In bevorzugten Ausgestaltungen wird entweder nur TiO₂ oder eine Mischung aus TiO₂ und ZrO₂ verwendet. Günstig ist ein Anteil von mindestens 7,5 Masse-%, stärker bevorzugt von mindestens 9,5 Masse-% an TiO₂. ZrO₂ kann in diesen Ausgestaltungen fehlen oder ebenfalls vorhanden sein.

Die Glaskeramik kann, muss aber nicht, weitere Zusätze wie Yttrium-, Lanthan-, Cer-, Germanium-, Tantal- und/oder Boroxid in einer Menge von bis zu ca. 15 Masse-% enthalten.

Bei der erfindungsgemäßen Glaskeramik handelt es sich vorzugsweise um eine rein oxidische Keramik. Das bedeutet, dass sie keine anderen Anionen außer O²⁻-Ionen enthält, ggf. mit Ausnahme von unbeabsichtigt eingeschleppten Verunreinigungen, die vorzugsweise jedoch nicht mehr als 1 Mol-% ausmachen, bezogen auf den Anteil an O²⁻-Ionen.

In bevorzugten Ausführungsformen besteht sie aus den vorgenannten Komponenten, mit Ausnahme möglicherweise unbeabsichtigt eingeschleppter Kationen. Insbesondere sollte sie frei sein von weiteren Alkali- und/oder Erdalkalüonen. Die Grenze hierfür liegt bei insgesamt ca. 1 Masse-%, bezogen auf die jeweiligen Oxide; vorzugsweise sind diese sowie weitere Kationen jedoch nur in ganz geringen Mengen (0,1 Masse-%, bezogen auf die Oxide) oder gar nicht vorhanden.

Erfindungsgemäß hat sich gezeigt, dass im verwendeten MAS-Hochquarzmischkristallsystem die gewählten Glaszusammensetzungen zu Glaskeramiken führen, die in einer Kristallisationszwischenstufe sehr gut bearbeitbar sind und im Endzustand hervorragende Festigkeitswerte, außerordentliche Transluzenz und deutlich erhöhte chemische Beständigkeiten aufweisen. Obwohl es hier nicht zu eindeutig zuordenbaren Phasenumwandlungen kommt, lässt sich das Material so vorkristallisieren, dass es sich mit einer kurzen thermischen Nachbehandlung von einer gut CAM-bearbeitbaren Zwischenstufe zu einem extrem hochfesten und transluzenten Endmaterial mit hervorragender chemischer Beständigkeit umwandeln lässt.

Zur Herstellung der erfindungsgemäß bearbeitbaren und anschließend hochfest gestalteten Glaskeramik wird aus den gewählten Komponenten zuerst ein Glas erschmolzen, was in der Regel bei 1500-1800°C gelingt. Nach Abkühlen auf Raumtemperatur erfolgt eine erste Kristallisationsphase, die ein- oder zweistufig ablaufen kann. Die Temperaturen hierfür liegen je nach individueller Materialzusammensetzung in der Regel im Bereich von 750 bis 880°C, können aber auch 900°C erreichen, wenn die Dauer der Behandlung, die üblicherweise bei ca. 20 min. bis 4 Stunden liegt, relativ kurz, nämlich bei nicht mehr als 2 Stunden, gehalten wird. Das Produkt dieser Phase ist sehr gut bearbeitbar.

Das bereits kristallisierte, ggf. wunschgemäß mechanisch bearbeitete Material bzw. Produkt wird sodann in einer zweiten Kristallisationsphase nachgehärtet und dabei in eine sehr feste Glaskeramik umgewandelt. Diese Härtung erfolgt bei höheren Temperaturen als denen der ersten Kristallisationsphase, nämlich bei mehr als 900°C und in der Regel bei ca. 925-1050°C für einen Zeitraum von bis zu 120 min, der kürzer als die erste Kristallisationsphase ist. Für diese zweite Härtung ist zu beachten, dass der Energieeintrag über die Temperatur und die Zeit nicht zu hoch sein darf: Wird die zweite und endgültige Härtung bei Temperaturen durchgeführt, die über einer gewissen Temperaturdifferenz gegenüber der der ersten Härtung liegen, oder wird die Dauer der zweiten Härtung zu lang gewählt, ist mit einer zunehmenden Bildung von Cristobalit zu rechnen, wodurch die Biegebruchfestigkeit der nachgehärteten Glaskeramik signifikant absinkt. Dies ist auf die dann leichtere Rissausbreitung zurückzuführen. Insbesondere sollte als Temperatur der zweiten Härtung eine solche gewählt werden, die zwischen 40K und maximal 150K, vorzugsweise maximal 100K über der der ersten Härtung liegt, während die Dauer in der Regel nur einen Bruchteil derjenigen beträgt, die für die erste Härtung eingesetzt wird, und höchstens halb so lang sein sollte.

Aufgrund der guten Bearbeitbarkeit der Glaskeramik nach dem ersten Temperschritt (der ersten Kristallisationsphase) und der sehr guten Härte, Rissfestigkeit und Transluzenz nach der Nachhärtung eignen sich die erfindungsgemäßen Glaskeramiken besonders gut als Dentalmaterialien, z.B. für dentale Restaurationen nach dem oben beschriebenen chair-side-Verfahren. In besonderen Ausgestaltungen dienen sie dabei als Dentalfarbe, wofür übliche Übergangsmetall-Kationen zugesetzt werden können, oder sie vermitteln einen leicht fluoreszierenden Eindruck, der der natürlichen Zahn-Fluoreszenz ähnlich ist. Dies gelingt durch den Zusatz von Kationen der Seltenerd-Elemente wie Europium, Terbium oder Praseodym.

### Ausführungsbeispiele

### Beispiel 1

Ein Glas wurde bei ca. 1650°C aus den folgenden Komponenten erschmolzen (Angaben in Masse-Anteilen (MA):

| | |
|---|---|
| SiO₂ | 41,5 |
| P₂O₅ | 8,0 |
| Al₂O₃ | 23,4 |
| MgO | 7,0 |
| TiO₂ | 5,7 |
| La₂O₃ | 12,6 |
| B₂O₃ | 1,8 |

Nach Abkühlen auf Raumtemperatur wurde das Material einer ersten Kristallisationsphase unterworfen, und zwar in zwei Schritten: Der erste Schritt erfolgte bei 780°C für 3 h und der zweite Schritt bei 850°C für 2 h. Es resultierte eine gut bearbeitbare Keramik mit einer 3-Punkt-Biegebruchfestigkeit von 150 MPa.

Diese wurden bei 975°C einer zweiten Kristallisationsphase (Härtungsphase) unterworfen. Die Kristallisation wurde variiert zwischen 60 und 120 min. Es resultierte ein Glaskeramik mit Festigkeiten von 310 bis 330 MPa (Einzelwerte > 410 MPa), guter Transluzenz und einer schlechten Rissausbreitung.

Als Kristallphasen ließen sich Hochquarzmischkristalle (ß-Quarz-Struktur) und Lanthanphosphat (LaPO₄) nachweisen.

### Vergleichsbeispiel 1

Beispiel 1 wurde wiederholt, wobei die zweite Kristallisationsphase (Härtungsphase) für 60 min bei 1000 °C durchgeführt wurde. Die Festigkeit betrug 160 MPa; im Material konnte Cristobalit als Kristallisationsphase nachgewiesen werden.

### Beispiel 2

Als ein weiteres Beispiel wurde ein dem Beispiel 1 ähnliches Glas bei ca. 1650°C aus den folgenden Komponenten erschmolzen (Angaben in Masse-Anteilen (MA), wobei das Titanoxid teilweise durch Zirkonoxid ersetzt wurde:

| | |
|---|---|
| SiO₂ | 40,7 |
| P₂O₅ | 7,8 |
| Al₂O₃ | 23,0 |
| MgO | 6,9 |
| TiO₂ | 1,9 |
| ZrO₂ | 5,7 |
| La₂O₃ | 12,3 |
| B₂O₃ | 1,8 |

Nach Abkühlen auf Raumtemperatur wurde das Material verschiedenen Kristallisationsprozessen unterworfen.

Wurde die erste Kristallisation für 2 h bei 900°C durchgeführt, ergab sich eine noch tolerierbare Festigkeit von 230 MPa für die Bearbeitungsphase. Mit einer Nachbearbeitung von 15 min bei 960°C ergab sich eine Biegebruchfestigkeit von 495 MPa (Einzelwerte > 600 MPa).

Als Kristallphasen ließen sich Hochquarzmischkristalle (ß-Quarz-Struktur) und Lanthanphosphat (LaPO₄) nachweisen.

### Beispiel 3 (Vergleichsbeispiel)

Als weiteres Beispiel wurde folgende Glaszusammensetzung erschmolzen:

| | |
|---|---|
| SiO₂ | 41,0 |
| P₂O₅ | 9,1 |
| Al₂O₃ | 23,1 |
| MgO | 7,0 |
| TiO₂ | 4,1 |
| ZrO₂ | 6,4 |
| La₂O₃ | 8,4 |
| B₂O₃ | 0,9 |

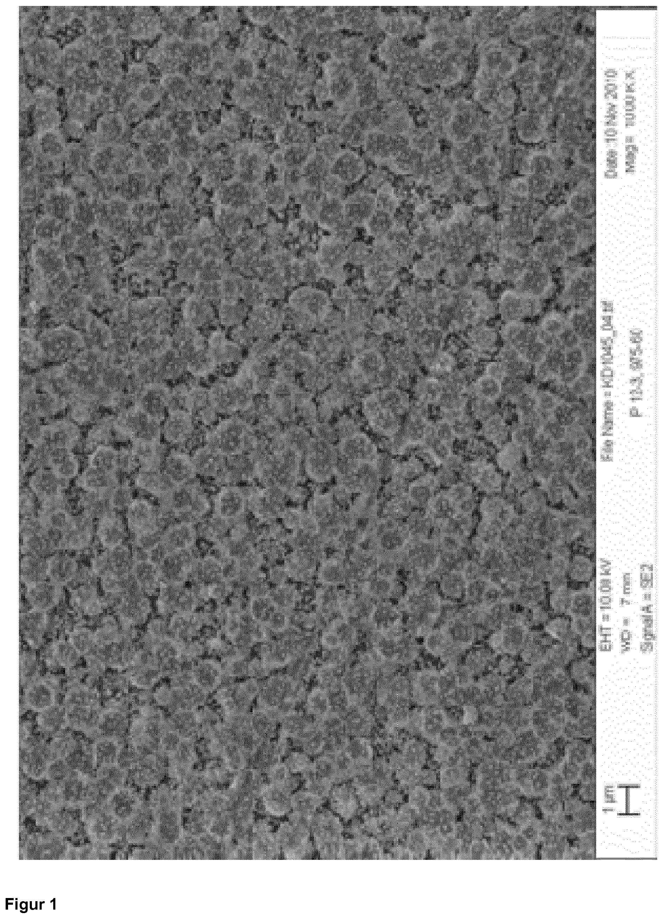

Nach Abkühlen auf Raumtemperatur wurde das Material einer ersten Kristallisationsphase von 930°C für 2 h unterworfen und anschließend bei 975°C für 60 min in der zweiten Kristallisationsphase auf die Endeigenschaften kristallisiert. Es resultierte ein Glaskeramik mit einer Festigkeiten von 445 MPa (Einzelwerte > 550 MPa), guter Transluzenz und einer schlechten Rissausbreitung. Als Kristallphasen wurden Hochquarzmischkristalle (ß-Quarz-Struktur), Rutil (TiO₂ und Lanthanphosphat (LaPO₄) nachgewiesen. Im Rasterelektronenmikroskop zeigt sich ein hoher Kristallisationsgrad mit Kristallen von ca. 1 µm Größe, siehe Figur 1.

### Beispiel 4

Ein Glas wurde bei ca. 1650°C aus den folgenden Komponenten erschmolzen (Angaben in Masse-Anteilen (MA):

| | |
|---|---|
| SiO₂ | 53,6 |
| P₂O₅ | 5,4 |
| Al₂O₃ | 17,9 |
| MgO | 13,6 |
| TiO₂ | 9,5 |

Nach Abkühlen auf Raumtemperatur wurde das Material einer ersten Kristallisationsphase unterworfen, und zwar in zwei Schritten: Der erste Schritt erfolgte bei 780°C und der zweite Schritt bei 850°C. Die Haltezeit lag jeweils zwischen 20 min. und 4 Stunden. Es resultierte eine gut bearbeitbare Keramik.

Diese wurde bei 950-1025°C einer zweiten Kristallisationsphase (Härtungsphase) unterworfen. Diese war deutlich kürzer als die der ersten Kristallisationsphase und lag zwischen 10 und 80 min. Es resultierte eine Glaskeramik mit sehr guter Festigkeit, guter Transluzenz und guten mechanischen Eigenschaften, insbesondere einer hohen Härte und einer schlechten Rissausbreitung.

### Beispiel 5

Beispiel 4 wurde mit der folgenden Glaszusammensetzung wiederholt:

| | |
|---|---|
| SiO₂ | 45,3 |
| P₂O₅ | 10,7 |
| Al₂O₃ | 25,6 |
| MgO | 7,7 |
| TiO₂ | 10,7. |

Die Ergebnisse waren vergleichbar.

### Beispiel 6

Beispiel 4 wurde mit der folgenden Glaszusammensetzung wiederholt:

| | |
|---|---|
| SiO₂ | 41,0 |
| P₂O₅ | 9,1 |
| B₂O₃ | 0,9 |
| Al₂O₃ | 23,1 |
| MgO | 7,0 |
| TiO₂ | 4,1 |
| ZrO₂ | 6,3 |
| La₂O₃ | 8,4. |

Die Ergebnisse waren vergleichbar.

## Patentansprüche

1. Verwendung eines Magnesiumoxid-Aluminiumoxid-Siliciumoxid-Glases oder einer solchen Glaskeramik zur Anwendung im Dentalbereich, wobei das Glas bzw. die Keramik weiterhin Phosphor und eine Übergangsmetallkomponente aufweist, ausgewählt unter Titan und Zirkonium oder einer Mischung aus beidem, umfassend die Schritte:
Erschmelzen eines Glases aus den entsprechenden Ausgangsmaterialien bei Temperaturen zwischen 1500 und 1800°C,
aktives oder passives Abkühlen des Glases auf Umgebungstemperatur, Bewirken einer ersten Kristallisation durch Erhitzen des Glases auf 750 bis <900°C für einen Zeitraum zwischen 20 min und 4 Stunden, oder 750°C bis 900°C für einen Zeitraum zwischen 20 min und 2 Stunden, wobei eine gut bearbeitbare Glaskeramik entsteht, aktives oder passives Abkühlen der so erhaltenen Glaskeramik auf Umgebungstemperatur,
Bewirken einer zweiten Kristallisation durch Erhitzen des Glases auf über 900°C für einen Zeitraum von bis zu 120 min, der jedoch kürzer ist als der Zeitraum, in dem die erste Kristallisation bewirkt wird,
**dadurch gekennzeichnet, dass** die durch die erste Kristallisation entstandene Glaskeramik mechanisch bearbeitet wird, wobei der Schritt des mechanischen Bearbeitens die Herstellung eines im Dentalbereich einsetzbaren Substituts, vorzugsweise einer Krone, eines Onlay oder eines Inlay, aus einem entsprechenden Rohling umfasst.

2. Verwendung nach Anspruch 1, worin die zweite Kristallisation für einen Zeitraum von nicht mehr als 80 min durchgeführt wird.

3. Verwendung nach Anspruch 1, worin die erste Kristallisation in zwei Stufen durchgeführt wird, wobei die erste Stufe bei 750-800°C, vorzugsweise bei ca. 780°C, und die zweite Stufe bei 820-880°C, vorzugsweise bei ca. 850°C erfolgt.

4. Verwendung nach einem der Ansprüche 1 bis 3, worin die zweite Kristallisation bei 950-1025°C erfolgt, mit der Maßgabe, dass die gewählte Temperatur nicht mehr als 150K über derjenigen der ersten Kristallisation liegt.

5. Verwendung eines Magnesiumoxid-Aluminiumoxid-Siliciumoxid-Glases oder einer solchen Glaskeramik nach einem der voranstehenden Ansprüche, **dadurch gekennzeichnet, dass** das Ausgangsmaterial ein reines Oxidglas ist.

6. Verwendung eines Magnesiumoxid-Aluminiumoxid-Siliciumoxid-Glases oder einer solchen Glaskeramik nach einem der voranstehenden Ansprüche, **gekennzeichnet durch** die nachstehende Zusammensetzung:
| | |
|---|---|
| SiO₂ | 30 - 60 MA |
| P₂O₅ | 3,5 - 15 MA |
| Al₂O₃ | 12 - 30 MA |
| MgO | 5 - 20 MA |
| Σ(TiO₂+ZrO₂) | 5 - 20 MA |
| Σ MₓO_{y} | 0 - 15 MA, |
wobei M ausgewählt ist unter Yttrium, Lanthan, Cer, Germanium, Tantal und Bor.

7. Verwendung eines Magnesiumoxid-Aluminiumoxid-Siliciumoxid-Glases oder einer solchen Glaskeramik nach Anspruch 6, **gekennzeichnet durch** die nachstehende Zusammensetzung:
| | |
|---|---|
| SiO₂ | 35 - 55 MA |
| P₂O₅ | 5 - 15 MA |
| Al₂O₃ | 16 - 27 MA |
| MgO | 6 - 14,5 MA |
| Σ(TiO₂+ZrO₂) | 5 - 15 MA |
| Σ MₓO_{y} | 0 - 12 MA. |

8. Verwendung einer Magnesiumoxid-Aluminiumoxid-Siliciumoxid-Glaskeramik nach einem der voranstehenden Ansprüche, worin die Hauptkristallphase ein Magnesium-Hochquarzmischkristall ist.

9. Verwendung einer Magnesiumoxid-Aluminiumoxid-Siliciumoxid-Glaskeramik nach einem der voranstehenden Ansprüche, umfassend eine Kristallphase aus Lanthanphosphat.

10. Verwendung nach einem der voranstehenden Ansprüche mittels CAD/CAM und/oder für Chair-side-Anwendungen.

11. Verwendung nach einem der voranstehenden Ansprüche, worin das Glas bzw. die Glaskeramik zusätzliche Übergangsmetallkationen aufweist und als Dentalfarbe eingesetzt wird.

12. Verwendung nach einem der voranstehenden Ansprüche, worin dem Glas bzw. der Glaskeramik zur Erzeugung einer die Natur nachahmenden Fluoreszenzwirkung ein Fluoreszenz-Zusatz beigegeben wird, vorzugsweise ausgewählt unter Kationen der Seltenerd-Elemente, besonders bevorzugt unter Er³⁺, Eu³⁺, Tb³⁺, Pr³⁺ und Gd³⁺.

## Claims

1. Use of a magnesium oxide-aluminum oxide-silicon oxide glass, or of such a glass ceramic, for application in the dental field, wherein the glass or the ceramic furthermore has phosphorous and a transition metal component, selected from titanium and zirconium or a mixture of the two, including the steps of:
fusing a glass from the corresponding initial materials at temperatures between 1,500 and 1,800 °C;
active or passive cooling of the glass to ambient temperature;
production of a first crystallization by heating the glass to 750 to <900 °C for a time period between 20 min and 4 hours, or 750 °C to 900 °C for a time period between 20 min and 2 hours, wherein a glass ceramic that can be processed well is created;
active or passive cooling of the glass ceramic obtained in such a manner to ambient temperature;
production of a second crystallization by heating the glass to over 900 °C for a time period of up to 120 min, which is, however, shorter than the time period in which the first crystallization is produced,
**characterized in that** the glass ceramic created via the first crystallization is mechanically processed, wherein the step of mechanical processing includes the production of a substitute - preferably a crown, an onlay, or an inlay - from a corresponding blank, which substitute can be replaced in the dental field.

2. Use according to claim 1, wherein the second crystallization is carried out for a time period of not more than 80 min.

3. Use according to claim 1,
wherein the first crystallization is carried out in two stages, wherein the first stage takes place at 750-800 °C - preferably, at approximately 780 °C - and the second stage takes place at 820-880 °C - preferably, at approximately 850 °C.

4. Use according to one of claims 1 through 3,
wherein the second crystallization takes place at 950-1,025 °C, with the stipulation that the selected temperature not be more than 150K above that of the first crystallization.

5. Use of a magnesium oxide-aluminum oxide-silicon oxide glass, or of such a glass ceramic according to any of the preceding claims, **characterized in that** the initial material is a pure oxide glass.

6. Use of a magnesium oxide-aluminum oxide-silicon oxide glass, or of such a glass ceramic according to any of the preceding claims, **characterized by** the following composition:
| | |
|---|---|
| SiO₂ | 30 - 60 MA |
| P₂O₅ | 3.5 - 15 MA |
| AI₂O₃ | 12 - 30 MA |
| MgO | 5 - 20 MA |
| ∑ (TiO₂+ZrO₂) | 5 - 20 MA |
| ∑MₓO_{y} | 0 - 15 MA |
wherein M is selected from yttrium, lanthanum, cerium, germanium, tantalum, and boron.

7. Use of a magnesium oxide-aluminum oxide-silicon oxide glass, or of such a glass ceramic according to claim 6, **characterized by** the following composition:
| | |
|---|---|
| SiO₂ | 35 - 55 MA |
| P₂O₅ | 5 - 15 MA |
| AI₂O₃ | 16 - 27 MA |
| MgO | 6 - 14.5 MA |
| ∑ (TiO₂+ZrO₂) | 5 - 15 MA |
| ∑MₓO_{y} | 0 - 12 MA |

8. Use of a magnesium oxide-aluminum oxide-silicon oxide glass ceramic according to one of the preceding claims, wherein the primary crystalline phase is a magnesium-beta quartz mixed crystal.

9. Use of a magnesium oxide-aluminum oxide-silicon oxide glass according to one of the preceding claims, comprising a crystalline phase made up of lanthanum phosphate.

10. Use according to one of the preceding claims by means of CAD/CAM and/or for chair-side applications.

11. Use according to one of the preceding claims, wherein the glass or the glass ceramic has additional transition metal cations and is used as a dental colorant.

12. Use according to one of the preceding claims, wherein a fluorescent additive is added to the glass or the glass ceramic to generate a fluorescence effect mimicking nature - preferably, selected from cations of rare earth elements, and, particularly preferably, from Er³⁺, Eu³⁺, Tb³⁺, Pr³⁺, and Gd³⁺.

## Revendications

1. Utilisation d'un verre d'oxyde de magnésium-oxyde d'aluminium-oxyde de silicium ou d'une telle vitrocéramique pour une application dans le domaine dentaire, le verre ou la céramique présentant en outre du phosphore et un composant de métal de transition, choisi parmi le titane et le zirconium ou un mélange des deux, comprenant les étapes de :
fusion d'un verre constitué par les matériaux de départ correspondants à des températures entre 1500 et 1800 °C,
refroidissement actif ou passif du verre à la température ambiante,
provocation d'une première cristallisation par chauffage du verre à 750 jusqu'à <900 °C pendant un laps de temps entre 20 min et 4 heures ou à 750 °C jusqu'à 900 °C pendant un laps de temps entre 20 min et 2 heures, une vitrocéramique pouvant être bien usinée se formant, refroidissement actif ou passif de la vitrocéramique ainsi obtenue à la température ambiante, provocation d'une deuxième cristallisation par chauffage du verre à plus de 900 °C pendant un laps de temps de jusqu'à 120 min, mais qui est plus court que le laps de temps pendant lequel la première cristallisation est provoquée,
**caractérisée en ce que** la vitrocéramique formée par la première cristallisation est usinée mécaniquement, l'étape d'usinage mécanique comprenant la fabrication d'un substitut utilisable dans le domaine dentaire, de préférence d'une couronne, d'un onlay ou d'un inlay, à partir d'une ébauche correspondante.

2. Utilisation selon la revendication 1, dans laquelle la deuxième cristallisation est réalisée pendant un laps de temps qui n'est pas supérieur à 80 min.

3. Utilisation selon la revendication 1,
dans laquelle la première cristallisation est réalisée en deux étapes, la première étape ayant lieu à 750-800 °C, de préférence à environ 780 °C et la deuxième étape ayant lieu à 820-880 °C, de préférence à environ 850 °C.

4. Utilisation selon l'une quelconque des revendications 1 à 3,
dans laquelle la deuxième cristallisation a lieu à 950-1025 °C, à condition que la température choisie ne soit pas supérieure de plus de 150 K à celle de la première cristallisation.

5. Utilisation d'un verre d'oxyde de magnésium-oxyde d'aluminium-oxyde de silicium ou d'une telle vitrocéramique selon l'une quelconque des revendications précédentes, **caractérisée en ce que** le matériau de départ est un verre d'oxyde pur.

6. Utilisation d'un verre d'oxyde de magnésium-oxyde d'aluminium-oxyde de silicium ou d'une telle vitrocéramique selon l'une quelconque des revendications précédentes, **caractérisée par la** composition suivants :
| | |
|---|---|
| SiO₂ | 30 - 60 parties en masse |
| P₂O₅ | 3,5 - 15 parties en masse |
| Al₂O₃ | 12 - 30 parties en masse |
| MgO | 5 - 20 parties en masse |
| ∑ (TiO₂+ZrO₂) | 5 - 20 parties en masse |
| ∑MₓO_{y} | 0 - 15 parties en masse, |
M étant choisi parmi l'yttrium, le lanthane, le cérium, le germanium, le tantale et le bore.

7. Utilisation d'un verre d'oxyde de magnésium-oxyde d'aluminium-oxyde de silicium ou d'une telle vitrocéramique selon la revendication 6, **caractérisée par la** composition suivante :
| | |
|---|---|
| SiO₂ | 35 - 55 parties en masse |
| P₂O₅ | 5 - 15 parties en masse |
| Al₂O₃ | 16 - 27 parties en masse |
| MgO | 6 - 14,5 parties en masse |
| ∑ (TiO₂+ZrO₂) | 5 - 15 parties en masse |
| ∑MₓO_{y} | 0 - 12 parties en masse. |

8. Utilisation d'une vitrocéramique d'oxyde de magnésium-oxyde d'aluminium-oxyde de silicium selon l'une quelconque des revendications précédentes, la phase cristalline principale étant un cristal mixte de magnésium-quartz bêta.

9. Utilisation d'une vitrocéramique d'oxyde de magnésium-oxyde d'aluminium-oxyde de silicium selon l'une quelconque des revendications précédentes, comprenant une phase cristalline en phosphate de lanthane.

10. Utilisation selon l'une quelconque des revendications précédentes par CAO/FAO et/ou pour des applications au fauteuil du dentiste.

11. Utilisation selon l'une quelconque des revendications précédentes, le verre ou la vitrocéramique présentant des cations supplémentaires de métal de transition et étant utilisé(e) comme colorant dentaire.

12. Utilisation selon l'une quelconque des revendications précédentes, dans laquelle le verre ou la vitrocéramique est additionné(e) d'un additif fluorescent pour générer un effet de fluorescence imitant la nature, de préférence choisi parmi les cations des éléments des terres rares, de manière particulièrement préférée parmi Er³⁺, Eu³⁺, Tb³⁺, Pr³⁺ et Gd³⁺.
